# EUROPEAN PATENT APPLICATION

(11) **EP 1 325 759 A1**
(43) Date of publication of application: **09.07.2003**
(21) Application number: 01960752.2
(22) Date of filing: 10.08.2001
(51) Int. Cl.: A61M 1/12

(54) **ARTIFICIAL INTRACAVITARY VENTRICLE**

(30) Priority: 10.08.2000 UY 2628900
(71) Applicant: Giambruno Marono, Juan Manuel, Montevideo (UY)
(72) Inventor: Giambruno Marono, Juan Manuel, Montevideo (UY)
(74) Representative: Maldonado Jordan, Julia
(86) International application number: ES0100321
(87) International publication number: WO02011789

(57) **Abstract**

An intracavity artificial ventricle (IAV), which comprises a mechanic circulatory assistance device, designed to be assembled inside the remaining and preserved muscular mass of a natural ventricle in terminal stage. Said design allows the use of the space available inside the anterior mediastinum, and preserves the irrigation and the function of the contralateral ventricle.

## Description

### FIELD OF THE INVENTION.

This invention pertains to medical prostheses, and in particular, to a mechanical circulatory assistance device. It has been conceived to be implanted in a novel orthotopic position, inside of a living being's heart. It satisfies the current need of finding an anatomic space to functionally substitute the native sick heart in its terminal stage or as a bridge to cardiac transplantation or to be used after a heart transplantation failure.

### BACKGROUND OF THE INVENTION.

At present, when there is a patient with a serious heart disease, which for different reasons is nonreversible, cardiac transplantation is considered as the solution. However, in the United States, for example, there are about 60,000 patients per year under this situation, and only about 6% to 10% get a transplantation due to the current difficulties to find an adequate heart donor. -----

The mechanical circulatory assistance devices are considered as a progress in case of such an extreme situation of a cardiac failure. The total artificial heart (TAH) is implanted in an orthotopic position, after the surgical resection of the muscular mass of both ventricles of the native heart, and these devices perform all blood pumping. Devices of partial circulatory assistance are also used, generally known as Ventricular Assist Devices (VAD). They are implanted in a heterotopic position, in some place of the patient's body, abdominal or thoracic region, acting in parallel or in series, they support or assist the failing native ventricle function. -

Under extreme haemodynamic failure circumstances, these circulatory assist devices allow to keep the patient alive while the patient awaits for the appropriate donor, preventing a serious systemic damage caused by the progressive deterioration of the haemodynamia, which can later compromise the viability of other organs if the patient gets a transplantation.

The present generation of mechanic circulatory assist devices has had problems. As a result of the anatomic discordance between the size of the present generation devices and the space available in the mediastinum for the current models, many of these devices do not place the artificial ventricles and some other necessary elements for its functioning inside the thorax. In some devices, different elements are located outside the body, and the joint of the internal and external parts to the human body is made through the skin. Many pathologic phenomena take place, such as local infections that later turn into more serious infections, ascending infections, skin ulcerations, and uncountable problems for the patient and his quality of life. At the same time, acting and putting elements outside the thoracic cavity causes surgical complications and complications during the post-surgical period, such as bleedings, hematological collections, infections and compressions.

Furthermore, due to the reduced space available within the chest, some of the devices of the present generation do not have an adequate size to produce a good final diastolic volume. Hence, often, in order to obtain an adequate blood flow rate, these devices resort to a significant increase of the heart frequency which causes additional turbulence as a result of an increase in the blood flow linear velocity. This situation can be the cause of more serious hematological complications, such as haemolysis and bleeding and the cause of a faster deterioration of the materials that form these devices. On these grounds, a better use of the space available inside the mediastinum to achieve a significant increase on the diastolic volume would be highly desirable.

The artificial devices present haemostatic complications such as bleeding. Different ventricular assist devices, as they are placed in an heterotopic position, far from the inlet and outlet holes of the blood to the native ventricle of the normal heart, to reach their artificial blood pumping chambers, the blood must go through long prosthetic tubes circuits. The artificial chambers of these devices do not receive the blood in their inlet port directly from the venous return, but they do it after it goes through the rigid prosthetic tubes, with stitches at each end. These artificial ventricles connect their outlet port to the corresponding artery, after it goes through prosthetic tubes. These artificial prosthetic tubes do not respond to the need of increasing the blood flow rate like native vessels do in reflex mode, i.e. by greatly increasing their diameter. This causes more turbulence and cell damages. This deficiency causes a larger increase in blood pressure which further stresses the above mentioned stitches and causes the present generation of artificial devices to operate under more stringent conditions.

As a record there is a device called Jarvik-7. The USPTO document n° 4,863,461 called Artificial Ventricle refers in the first line of the abstract to: "An artificial ventricle for replacing the human heart... " .In column 2 line 24-26 refers to: "... provides such an artificial ventricle as can be combined with second, similar ventricle in a replacement for the human heart ...". In column 9 line 49-51, refers to: "...with the left ventricle resting deeply within the chest and right ventricle more anteriorly to its side". The following lines 52 and 53 refer to: "... a pair of such ventricles joined together...".

### SUMMARY OF THE INVENTION.

In general, the present invention comprises an intracavity artificial ventricle to be implanted in an orthotopic position in a living being, e.g. a human being, inside the remaining and preserved muscular mass of the failing native ventricle. This intracavity artificial ventricle is essentially constituted by an intracavity artificial blood chamber, a compressing mechanism and fixing means to the remaining and preserved muscular mass of the failing native ventricle. Thus, the structure of the intracavity artificial ventricle, with its fixing means, achieves an orthotopic position as a way to impel the blood, to substitute only the function of one of both native ventricles. This is how this intracavity artificial ventricle achieves anatomical fit.

The intracavity artificial ventricle on its external side has a configuration, shape and surface, constituted by holding elements and functional support, and by fixing means, in a non-traumatic and haemostatic way, with the remaining and preserved muscular mass of the failing native ventricle.

The improvement of the preservation of all the remaining muscular mass of the failing native ventricle, together with the rest of the muscular mass of the heart, is essential for the preservation of the coronary irrigation of the native contralateral ventricle functionally preserved.

This invention presents the improvement that the space volume occupied by the intracavity artificial ventricle in the dysfunctioning cavity is in closed contact with the septum, and stands the distention or protrusion of the septal wall, done by the other preserved and functioning ventricle towards the other side. It is the support of the important compressing function of the septal wall of the functionally preserved native ventricle, and all its muscular mass, and then, all together, the intracavity artificial ventricle and the action of all the remaining and preserved muscular mass of the native failing ventricle, support the pumping function of the blood of the other functionally preserved native ventricle.

The volume of the intracavity artificial ventricle is total or partially included in the remaining and preserved muscular mass of the failing native ventricle. In some of its functioning variations, the intracavity artificial ventricle is constituted by a single piece, including the compressing mechanism.

The intracavity artificial ventricle presents an intracavity artificial blood chamber, inside which the blood circulates. The intracavity artificial blood chamber, in an orthotopic position has an inlet port for the blood to enter directly from the corresponding auricle, or from some sector of the corresponding venous return, and an outlet port with means of connection to send the blood to the corresponding main artery. Said outlet port includes or it is adjacent to its main artery valve. A variation of the intracavity artificial blood chamber has means of attachment to some sector of the remaining and preserved dysfunctional muscular mass of the failing native ventricle, i.e. the outlet pathway. This variation of the intracavity artificial ventricle, has the intracavity artificial blood chamber continuing part of the cavity of the ventricle that functionally supports. This variation has means to attach to the remaining and preserved muscular mass of the failing native ventricle, at outlet pathway level. Another variation has the special characteristic that the intracavity artificial ventricle is constituted by an intracavity artificial blood chamber of only one movable artificial wall, with means of fixing to the free wall of the left ventricle, together with its compressing mechanism. It has means of attachment to the remaining and preserved muscular mass of the left ventricle, in front of the papillary anteroseptal muscle and outside the papillary anterolateral muscle, and it does not present this particular intracavity artificial blood chamber neither an inlet port nor an outlet port. This variation presents the enormous advantage that if the rest of the remaining and preserved muscular mass anatomy of the left ventricle allows it, it achieves the conservation of the inlet valve or mitral valve of the left failing ventricle. Remodeling ventricle criteria are considered for example for the lateral opening of the muscular mass of the failing left ventricle.

The compressing mechanism of the intracavity artificial ventricle, for the introduction and expelling of the blood from and towards the circulatory torrent, is electrohydraulic, electromechanic, pneumatic, muscular or biologic, rotating or centrifugal, etc.

This spatial arrangement of the intracavity artificial ventricle of the present invention achieves an excellent usage of the disposable space inside the mediastinum. It has the corresponding advantage of preserving the direct space relation of the blood connections of the substituted natural ventricle, and the use of the interior space of the failing native ventricle to place there the compressing mechanism. In addition, its inlet port and outlet port receive and send the flow of the blood to the circulatory system without the interposition of long artificial prosthetic tubes.

Many significant improvements are achieved through the present invention:
- 1-: In the preferred electrohydraulic embodiment and in other variations of the present invention, the intracavity artificial ventricle together with its compressing mechanism, is a single piece structure, completely located inside the thoracic cavity. ---
- 2-: As it preserves the muscular mass of the native ventricle to be functionally substituted, it allows the irrigation and correct blood pumping function of the preserved contralateral ventricle. -------
- 3-: It does not use prosthetic tubes, as the intracavity artificial blood chamber receives blood directly through its inlet port, from the corresponding vessel return, and impels it directly towards its main corresponding artery.
- 4-: A variation of the intracavity artificial blood chamber presents the important improvement of preserving the native inlet valve, mitral valve of the left ventricle.

An important haemodynamic and hematological advantage of the present invention is that, by placing the blood chambers' outflow near the systemic and pulmonary vascular regions, less stitches are needed, and this characteristic provides the present invention with the great advantage of being directly connected to the vascular systems through native vessels which respond to an increased blood flow with the vessel dilator autonomous reflex response. Hence, no increased pressures are needed to get a higher blood flow, thereby reducing the pressure on the walls of the blood chambers and the turbulence and associated blood stress, all of which greatly reduces the subsequent damage that this causes to blood cells and to the life of this new heart pumping system.

The intracavity artificial ventricle of the present invention is one-piece artificial blood chamber, or partial native cardiac muscular mass and partial prosthetic material, biologic or not, that have non-thrombogenetic characteristics in the surface which is in contact with the blood of soft and flexible walls, such as pericardium or silicon. In addition, for example in the electrohydraulic variation of the present invention, the cellular damage is reduced because the blood is pumped by the action of a homogeneous force distributed in a concentric manner.

This intracavity artificial ventricle has another different functional variation, in which the discharging blood expel is done by a rotating mechanism of centrifugal pump, having the intracavity artificial blood chamber rigid or semi-rigid walls, and is constituted, for example, by a dragging rotating cone.

Additional objects and concomitant advantages of the present invention will be set forth, in part, in the following description, or may be learned from practicing or using the present invention. The objects and the advantages may be achieved and attained by the means, features and/or combinations particularly pointed out in the claims attached hereto. It is understood that the foregoing general description and the following detailed description are exemplary and explanatory only and are not to be viewed as being restrictive of the invention as claimed.

### BRIEF DESCRIPTION OF THE DRAWINGS.

The accompanying drawings, which are herein incorporated and constitute a part of the specification, illustrate embodiments of the present invention and, together with the description, serve to explain the principles of the present invention.
**Fig. 1** View of a front section of the normal natural non dilated ventricle of a human being.
**Fig. 2** Front section that shows the implanting of the electrohydraulic variation of the intracavity artificial ventricle inside the dilated cavity of the left natural ventricle of a human being, in haemodynamic failure.
**Fig. 3** View of a cross section of the natural normal non-dilated ventricle of a human being, which runs at valve level.
**Fig. 4** Cross section that shows the implanting of the electrohydraulic variation of the intracavity artificial ventricle, inside the dilated cavity of the left natural ventricle in haemodynamic fail of a human being

### DETAILED DESCRIPTION OF THE INVENTION.

This invention is herein described in detail, as a non-limiting model and as the preferred way to develop it at present. It is also illustrated in the pictures attached hereto.

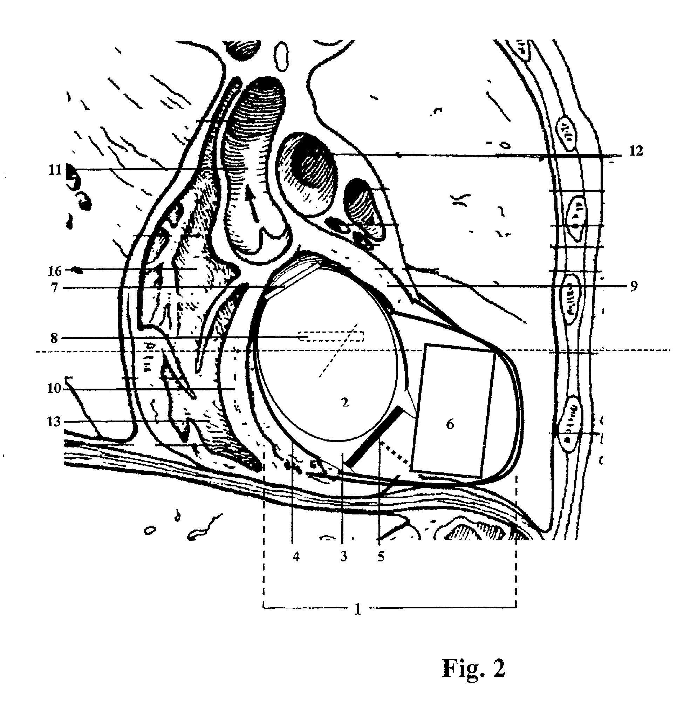

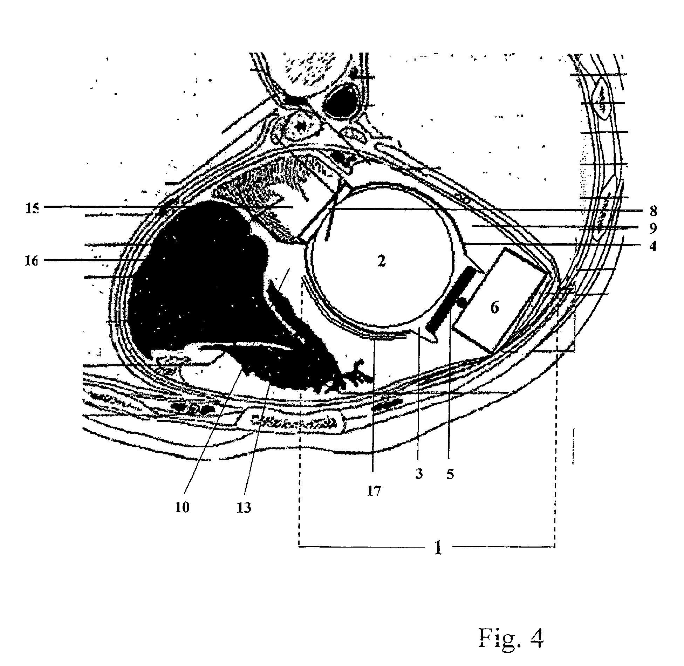

At present, the specific and preferred way to build the artificial intracavity ventricle **1**, according to this invention, is the one illustrated as a model in Fig. 2 and 4. Notwithstanding it, the present invention may be subject to different shape, size and material modifications, and the present specifications are not intended to limit the invention to the particular shapes, sizes and materials herein described. On the contrary, the intention is to cover all modifications and alternative executions that are within the spirit and the purpose of the invention in accordance with the claims attached hereto.

Moreover, as there shall be several modifications and changes that shall be analyzed by the experts in this field, we do not wish to limit the invention to the exact construction or operation described herein. Therefore, any and all equivalent modifications shall be considered as included within the scope of the present invention.

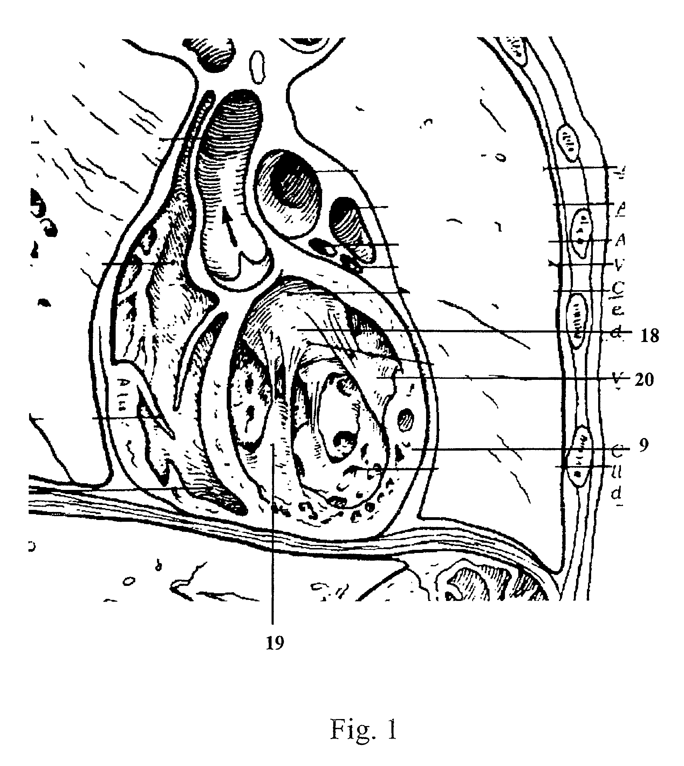

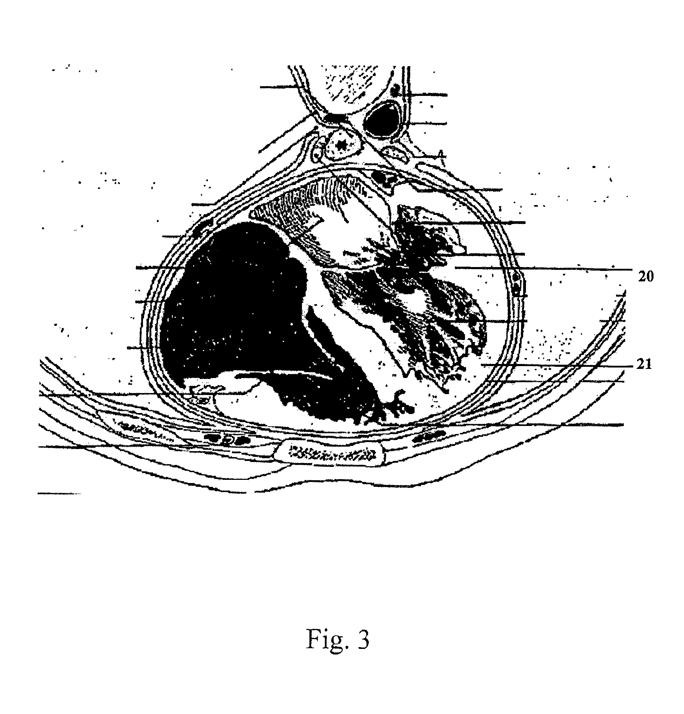

In fig. 1 and 3, an anatomical front and cross section of the natural non dilated cavities of the normal heart of a human being is observed

The preferred design of the intracavity artificial ventricle **1** of the present invention is that in which the pumping action of the blood is made by an electrohydraulic mechanism, as observed in fig. 2 and 4. The outside surface of the intracavity artificial ventricle **1** has means of attachment to the remaining and preserved muscular mass **9** of the failing native ventricle. Its interior is formed by an external compressing chamber **4**, which presents at least one movable surface **5**, by the intracavity artificial blood chamber **2**, by a compressing fluid **3**, and by the compressing mechanism **6**. This intracavity artificial ventricle **1** is also formed by other components, which are placed inside or outside the body: physiologic sensors, control panel, battery, etc.

The self-adaptable fixing system of the intracavity artificial ventricle to the ventriculotomy of the remaining and preserved muscular mass **9** of the failing native ventricle, has three parts. An adherent mesh over the intracavity artificial ventricle with a rough cover, Wooven-Dacron, Haemagill, for example, or a similar one. A fixed nerve or edge between 5 to 10 millimeters high, on the opposite margin of the outlet and inlet ports, with a base extension to the cardiac point, i.e., which is made of metal or of a synthetic material. It is the support of the free mesh. This free mesh, with a 50 millimeters extension at each side of the nerve or edge is made of dacron, for example. Its length allows the adaptation to the free border of the remaining and preserved muscular mass **9** of the native failing ventricle, adapting it to the fixing requirements of each case. Another variation of the fixing system of the intracavity artificial ventricle **1**, for example, presents an external surface with multiple walls **17**. One more internal, adherent, as the one already described, intermediate of goretex, for example, as a half support and sliding with a lubricant material, i.e, graphite, connected between themselves and with one more external, with non-traumatic and haemostatic fixing means, with the remaining dysfunctional mass **9** of the natural ventricle to be functionally substituted.

The intracavity artificial ventricle **1** to be implanted to functionally substitute the failing left ventricle when it presents the variation of the intracavity artificial blood chamber which has its outlet port **7** at the ring level of the aortic valve has an external compressing chamber **4** with an external slightly oval shape, as seen in Fig. 2 and Fig. 4. Its shape agrees with the interior shape of all the dysfunctional cavity to be occupied. It presents a lateral structure corresponding to its compressing mechanism **6**.

This intracavity artificial ventricle **1** presents an intracavity artificial blood chamber **2**, which is a sac of one or more soft and flexible walls, created to pump the blood. This one is placed totally or partially inside the remaining and preserved muscular mass **9** of the native ventricle in terminal haemodynamic failure, dilated or not, before the corresponding auricle position, in connection with the corresponding main artery; having then the running of its outlet pathway the same space disposition as the natural one. Its internal wall is constituted by a single piece of pig pericardium, for example. This internal wall could also be synthetic, i.e. of silicon.

The intracavity artificial blood chamber **2**, in its different variations, has a rear inlet port **8**, as it is shown in Fig. 4, for the blood to enter directly from the corresponding auricle, the left auricle **15**, or the right auricle **16**, or from some sector of the corresponding venous return. This inlet port **8** contains an unidirectional entrance valve included in a quick connector. A variation of the intracavity artificial ventricle **1**, implanted in functional substitution of the left native ventricle in failure, in which the intracavity artificial blood chamber **2** is made only for a wall, allows the preservation of the mitral valve itself **18**, for example, or the inlet port has a prosthetic valve. The intracavity artificial blood chamber **1**, has an outlet port **7**, with connection means to eject the blood to the corresponding main artery, aorta artery **11** or pulmonary artery **12**. This outlet port of the intracavity artificial blood chamber **2** includes or is adjacent to the valve of its main artery and has means of holding the valve ring, keeping the natural valve, or presents a prosthetic unidirectional valve, included in a quick connector. In a variation of the intracavity artificial ventricle **1**, the intracavity artificial blood chamber **2** has its outlet port with means to be joint to the outlet pathways of the native ventricle or other place of the remaining muscular mass **9** of the native ventricle in failure. This variation of the intracavity artificial blood chamber does not reach in this case the valve level of the corresponding main artery, and it is a mixed one, partially a synthetic wall and partially a preserved and remained muscular mass **9** of the native ventricle in failure.

A variation of the intracavity artificial ventricle **1** presents an intracavity artificial blood chamber **2** which only has an artificial wall, and this wall is a movable one. It has means of attachment to the free wall of the left ventricle **21**, for example, in front of the anteroseptal papillary muscle **19** of the sick natural left ventricle, and outside of the rear side papillary muscle **20**. The attachment is done with a quick-connection stitching or directly to the preserved and remaining muscular mass **9** of the native ventricle in failure. The wall of the intracavity artificial blood chamber **2** comprises in part, i.e., an internal surface of the preserved and remaining muscular mass **9** and in part a prosthetic material. It is semi-rigid in order to receive the electromechanical direct action or, for example, the pericardium or a synthetic material for the electrohydraulic mechanical action. This allows for the preservation of the integrity of all the mitral valve apparatus, with its native valve, which the anterior leaflet **18** is observed in figure 1. It has, for example, an electromechanic, electrohydraulic, muscular or a pneumatic compressing mechanism.

The intracavity artificial ventricle **1** has an compressing mechanism **6**, placed inside the pericardium or thoracic cavity for the electrohydraulic preferred design and for the electromechanic design. It is also intra-thoracic the action of a muscle surgically removed of its natural insertion, or by the action of a centrifugal mechanism. The intracavity artificial ventricle presents a variation in which the expelling function of the blood is produced through a pneumatic mechanism. The pneumatic impeller mechanism, and the electrohidraulic and centrifugal ones, in a functional variation, are placed outside the thoracic cavity.

The external compressing chamber **4**, as shown in Fig. 2 and 4, has inside it the intracavity artificial blood chamber **2** already described, and counts with two openings, inlet and outlet, which agree and have a sealed attachment to the inlet port **8** and the outlet port **7** of the intracavity artificial blood chamber **2**. It presents one or more movable surfaces **5**.

The compressing fluid **3** (for example glycerin), as shown in the schematic representation of Fig. 2 and 4, occupies the volume defined by the internal side of the external compressing chamber **4**, the movable surface **5** and the wall of the intracavity artificial blood chamber **2** constituting a sealed space. This compressing fluid **3** is used to transfer the impelling force of the movable surface **5** to the external wall of the intracavity artificial blood chamber **2**. The compressing fluid **3** contained inside the external compressing chamber **4**, acts in such a way that when the movable surface **5** has a filling or a diastolic position, allows the intra cavity artificial blood chamber **2** to reach its diastolic filling volume, when the blood enters through the inlet port **8**, and has its outlet port **7** closed. It has a volume, for example, of 40 to 90 cubic centimeters.

When the movable surface **5** moves inside the external compressing chamber **4**, this moving surface **5** reaches the blood ejection position or systolic position, and transfers the forces received from the compressing mechanism **6** to the compressing fluid **3**, which compresses the wall of the intracavity artificial blood chamber **2**, producing the emptiness effect of its internal volume, getting in such a way the expulsion or ejection of the blood contained inside the intracavity artificial blood chamber **2**, through the outlet port **7**, having its inlet port **8** closed. The moving surface **5** has a diameter area of five, six or more centimeters, and makes a movement of two, three or more centimeters, for example

The pneumatic design of the intracavity artificial ventricle **1**, is a variation of the electrohydraulic design, outlined in Fig. 2 and 4, in which the external compressing chamber **4**, that surrounds the intracavity artificial blood chamber **2** of this pneumatic variation, is the external compressing chamber **4** of the electro hydraulic variation, but the difference is that its walls are not movable, and has an additional opening. Through it, it connects to a source, for example, a tube coming from outside the patient's thorax, by which an external pneumatic console introduces and extracts a non-compressible fluid, for example a gas as the helium is. This external compressing chamber **4** of the pneumatic variation is characterized for their semi-rigid walls with a very little change in the volume when varying the internal pressure through injection and extraction of gas, for example. Inside, it contains the same intracavity artificial blood chamber **2** already described. To produce the compressing effect on the intracavity artificial blood chamber **2** of the pneumatic variation, a change in the volume of the compressing fluid is done **3**, for example, by injection and extraction of gas inside the external compressing chamber **4** of the pneumatic variation.

The electromechanic design of the intracavity artificial ventricle **1** is a variation of the electrohydraulic design outlined in Fig 2 and 4, in which the pumping function of the blood from the intracavity artificial blood chamber **2** of the electromechanic variation, is done by a compressing mechanism **6** acting directly on the wall or walls of the intracavity artificial blood chamber **2**. The intracavity artificial blood chamber **2** is fully artificial, having one inlet port and one outlet port, with parallel walls on which one or two movable surfaces **5** are supported. Also a variation in the constitution of the intracavity artificial blood chamber **2**, for the electromechanic operation, is that in which the intracavity artificial blood chamber **2** is partly made by the preserved and remaining ventricular mass **9** of the native ventricle in failure, and by one artificial wall, which is movable by the compressing mechanism. The intracavity artificial ventricle has fastening means in the outlet pathway or in the free wall **21** of the failing natural ventricle. The compressing mechanism **6** moves the movable surface **5**, which acts directly on the artificial wall of the intracavity artificial blood chamber **2** of this electro mechanic variation, to get the effect of blood pumping.

A muscular variation of the design of the intracavity artificial ventricle **1** is that the expelling of the blood action is produced by the action of a muscle, for example, latis dorsalis. This muscle is surgically removed from one of its extremes of its natural insertion and is taken inside the corresponding hemi thorax, and has fastening means; for example, by the inside, the outside, or in continuity with the edge of the preserved and remaining muscular mass of the functionally substituted ventricle, or surrounding one of the variations of the intracavity artificial blood chamber **2** already described, or a variation of it, for example, where the intracavity artificial blood chamber **2** is more elongated on its sides. The artificial electric stimulation of this muscle comprises the intracavity artificial blood chamber **2**, producing the expelling of the blood from the inside.

A muscular variation, for example, is that in which the muscle surrounds the opening of the free wall **21** of the opening cardiac muscular mass of the natural failing ventricular cavity. This opening is covered or not by a prosthetic or synthetic level, for example, the pericardium. All this mixed structure, the preserved and remaining muscular mass **9** and the synthetic one forms the intracavity artificial blood chamber 2 and is operated by this muscular impelling mechanism, and also receives the action of the remaining muscular mass, for the blood expelling.

Another variation of the way this muscle acts in order to expel the blood of the intracavity artificial blood chamber **2** is the connection of the latis dorsalis muscle, for example, which has means to fasten and start the movable surface **5**, of the external compressing chamber **4** of the described device for the electrohydraulic variation, and to start the movable surface **5**, of the previously described electro mechanic variation.

Another variation in the design of the intracavity artificial ventricle **1** is that in which the mechanism to impel the blood is a centrifugal pump. In this case, the intracavity artificial blood chamber **2** is a dragging rotating cone, for example, of rigid or semi-rigid walls and the intracavity artificial ventricle **1** expels the blood with continuous or pulse flow. This intracavity artificial blood chamber of centrifugal variation or dragging rotating cone is disposed in such a way that its plane face or base, by which it receives the impelling rotating movement is directed obliquely, to the sides and somewhat forwards, in connection to the electric rotating impelling mechanism, placed laterally, for example, making a single piece structure. The inlet and outlet port of this variation of the intracavity artificial blood chamber or dragging rotating one have ways to connect itself to the hole of the corresponding circulatory system, and these inlet and outlet doors have ways of connection to reach the necessary inclination to achieve the connections with the auricle or with some sector of the corresponding vessel return, and with the main corresponding artery. Another variation of the position of the dragging rotating cone proposed or intracavity artificial blood chamber of the centrifugal variation is that which has its plain face or base directed to the front, on the anterior costal wall. The electric rotating impelling mechanism in this centrifugal variation is outside the thoracic cavity, for example, transmitting its action in an electromagnetic way, going through the thorax wall. It may include or not, the resection of one or more ribs, or part of the sternum.

Another variation in the design of this intracavity artificial ventricle **1**, allows us to simultaneously assemble an intracavity artificial ventricle to substitute the pumping function of the blood, of each one of both native ventricles, simultaneously.

Many equivalents to the specific performing of the invention described herein will be identified or tested by simple routine experimentation. The intention is to include those equivalents in the scope of the following claims

## Claims

1. An intracavity artificial ventricle to be implanted totally or partially inside the cavity of the left ventricle or of the right ventricle of the native heart of a living being with a functional failure, preserving the remaining muscular mass, and comprising:
- an intracavity artificial blood chamber having an inlet port that receives the blood, with means of attachment to the auricle-ventricle hole or to some sector of the corresponding venous return, and an outlet port having means of connection to send the blood to the corresponding main arterial system,
- a compressing mechanism to be located inside the thoracic cavity,
- said intracavity artificial ventricle is **characterized by** its external surface which has means of attachment and support to the preserved and remaining muscular mass of the native ventricle functionally substituted

2. An intracavity artificial ventricle as defined in claim 1, in which the intracavity artificial blood chamber has one or more soft and flexible walls.

3. An intracavity artificial ventricle as defined in claim 1, in which the intra cavity artificial blood chamber has means of attachment to the corresponding main artery or to its outlet pathway.

4. An intracavity artificial ventricle as defined in claim 1, in which the intracavity artificial blood chamber is made by one artificial wall, which is movable, and by the preserved and remaining muscular mass of the native ventricle functionally substituted.

5. An intracavity artificial ventricle as defined in claim 4, with the preservation of the native auricle-ventricle valve of the natural ventricle functionally substituted.

6. An intracavity artificial ventricle as defined in claim 1, comprising:
- a compressing mechanism to be located inside the mediastinum to fulfill both functions, the expansion and the contraction of the intracavity artificial blood chamber.

7. An intracavity artificial ventricle as defined in claim 6, in which said compressing mechanism comprises:
- an external compressing chamber that encloses the intracavity artificial blood chamber, said external comprising chamber has one or more movable surfaces, and two or more openings, each of which coincides with the inlet and outlet port of the intracavity artificial blood chamber,
- a comprising fluid that fills the space between said external compressing chamber and the intracavity artificial blood chamber, said compressing fluid transmits the strength from the movable surface or surfaces of said external compressing chamber to make the expansion and the contraction of said intracavity artificial blood chamber,
- a power source to be located inside the mediastinum that moves one or more moving surfaces.

8. An intracavity artificial ventricle as defined in claim 1, in which said compressing mechanism comprises:
- an external compressing chamber with semi-rigid walls, said external compressing chamber encloses the intracavity artificial blood chamber; said external compressing chamber has three or more individual openings, two of said openings coincide, each of them, with the corresponding inlet and outlet port of the intracavity artificial blood chamber,
- a compressing fluid that fills the space between said external compressing chamber and the intracavity artificial blood chamber.
- means to connect said external compressing chamber to a source to add said compressing fluid; said compressing fluid makes the expansion and the contraction of the intracavity artificial blood chamber; said expansion and contraction to be effected by removing and inserting said compressing fluid from and in said compressing chamber, respectively.

9. An intracavity artificial ventricle as defined in claim 6, in which said compressing mechanism comprises:
- one or more movable surfaces, in direct contact with the intracavity artificial blood chamber, said rigid or semi-rigid movable surface or surfaces make the expansion and contraction of the intracavity artificial blood chamber,
- a power source to be located inside the mediastinum to move said movable surface or surfaces.

10. An intracavity artificial ventricle as defined in claim 1, in which the compressive mechanism is a muscle.

11. An intracavity artificial ventricle as defined in claim 1, having the intracavity artificial blood chamber rigid or semi-rigid walls, and a centrifugal impelling mechanism.
